(19) Europäisches Patentamt
European Patent Office
Office européen des brevets

(11) EP 1 104 751 A1

(12) # EUROPÄISCHE PATENTANMELDUNG

(43) Veröffentlichungstag:
**06.06.2001 Patentblatt 2001/23**

(51) Int Cl.[7]: **C07C 205/06**, C07C 201/08

(21) Anmeldenummer: **00125282.4**

(22) Anmeldetag: **21.11.2000**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **03.12.1999 DE 19958389**

(71) Anmelder: **BAYER AG**
**51368 Leverkusen (DE)**

(72) Erfinder:
- **Gürtler, Christoph, Dr.**
  **50676 Köln (DE)**
- **Jautelat, Manfred, Dr.**
  **51399 Burscheid (DE)**
- **Schelhaas, Michael, Dr.**
  **50733 Köln (DE)**

(54) **Verfahren zur Herstellung eines Dinitronaphthalin-Isomerengemisches mit erhöhtem Anteil an 1,5-Dinitronaphtalin**

(57) Die vorliegende Erfindung betrifft ein Verfahren zur Herstellung eines Dinitronaphthalin-Isomerengemisches mit einem erhöhtem Anteil an 1,5-Dinitronaphthalin durch Nitrierung von Naphthalin in Gegenwart mindestens einer ionischen Flüssigkeit.

EP 1 104 751 A1

## Beschreibung

Die Erfindung betrifft die Herstellung eines Dinitronaphthalin-Isomerengemisches mit erhöhtem Anteil an 1,5-Dinitronaphthalin durch direkte Nitrierung von Naphthalin. 1,5-Dinitronaphthalin ist eine Schlüsselverbindung für die Herstellung von 1,5-Diaminonaphthalin. Dieses ist u.a. die Ausgangsverbindung zur Herstellung von 1,5-Diisocyanatonaphthalin (Handelsnamen: Desmodur 15®). 1,5-Diisocyanatonaphthalin wird als Isocyanatkomponente bei der Polyurethanherstellung eingesetzt.

Die Herstellung von nitrierten Aromaten ist seit langem bekannt (G.A. Olah et al., Nitration: Methods and Mechanism, VCH, New York, 1989). Seit Jahrzehnten werden entsprechende Nitroaromaten durch Nitrierung mit einer Mischung aus Schwefel- und Salpetersäure (sogenannte Misch- oder Nitriersäure) technisch hergestellt.

Die Nitrierung von Naphthalin in Mischsäure liefert bei erhöhten Temperaturen von 80 bis 100°C ein Gemisch verschiedener Dinitronaphthaline. Dieses Gemisch besteht hauptsächlich aus 1,5- und 1,8-Dinitronaphthalin im Verhältnis von etwa 1 zu 2. Darüber hinaus enthält das Gemisch etwa 5 % andere Isomere, beispielsweise 1,6-und 1,7-Dinitronaphthalin. Die ungünstige Selektivität der Reaktion führt also dazu, dass bei der Herstellung von 1,5-Dinitronaphthalin vorzugsweise ein hoher und unerwünschter Anteil an 1,8-Dinitronaphthalin entsteht.

In DE-OS 11 50 965 wird die Herstellung von 1,5-Dinitronaphthalin ausgehend von 1-Nitronaphthalin beschrieben. Eine Erhöhung der Selektivität zugunsten des gewünschten 1,5-Isomeren wird durch schnelles und intensives Vermischen des in Schwefelsäure gelösten 1-Nitronaphthalins mit Nitriersäure erreicht. Nachteil dieses Verfahrens ist die erhebliche Menge an Schwefelsäure und deren aufwendige und kostenintensive Aufarbeitung. Außerdem können bei diesem Verfahren erhebliche Mengen an trinitrierten Produkten entstehen, die sowohl die 1,5-Dinitronaphthalinausbeute deutlich mindern, als auch im Bezug auf die Sicherheitstechnik als kritisch anzusehen sind, insbesondere bei der im genannten Stand der Technik beschriebenen adiabatischen Reaktionsführung.

In WO 99/12887 wird - ebenfalls ausgehend von 1-Nitronaphthalin - ein Verfahren zur Herstellung eines Dinitronaphthalin-Isomerengemisches mit erhöhtem Anteil an 1,5-Dinitronaphthalin beschrieben. Hierbei findet die Umsetzung des Nitronaphthalins mit Salpetersäure in Gegenwart eines festen, perfluorierten, stark sauren Ionenaustauschers statt. Generell hat dieses Verfahren den Nachteil, dass das entstandene Dinitronaphthalin-Isomerengemisch durch Extraktion mit Dioxan bei 90°C vom Katalysator abgetrennt werden muss. Das Dioxan muss anschließend durch einen zusätzlichen Destillationsschritt entfernt werden. Die besten Selektivitäten hinsichtlich des gewünschten 1,5-Isomers werden bei Zusatz von Sulfolan oder Nitromethan als Lösungsmittel erhalten. Der Umsatz der Reaktion liegt dann allerdings nur bei 31 bis 44 % und es entstehen beträchtliche Anteile anderer Dinitroisomere und trinitrierter Naphthaline, die schlechter aus dem Isomerengemisch abzutrennen sind.

DE-OS 24 53 529 beschreibt ausgehend von Naphthalin die Herstellung von Dinitronaphthalinen durch Nitrierung mit Salpetersäure in organischen Lösungsmitteln, beispielsweise Dichlorethan, und azeotroper Entfernung des Reaktionswassers. Mit diesem Verfahren wird Dinitronaphthalin in hohen Ausbeuten erhalten, ohne jedoch das Isomerenverhältnis zu beeinflussen.

WO 94/19310 beschreibt Nitrierungen von Nitroaromaten an mit teilweise Schwermetall-dotierten Aluminiumsilikaten, sogenannten Claycops, als festen Katalysator, die hohe Ausbeuten an dinitrierten Produkten bei geringen Mengen an Trinitroaromaten liefern. Nach diesem Verfahren durchgeführte Nitrierungen von Naphthalin liefern allerdings Isomerenverhältnisse wie bei klassischen Nitrierungen mit Mischsäure.

Es bestand daher die Aufgabe, ein Verfahren zur Herstellung eines Dinitronaphthalin-Isomerengemisches durch Nitrierung von Naphthalin bereitzustellen, bei dem bei gleichzeitigem hohen Umsatz und geringem Anteil an Nebenprodukten ein Gemisch von 1,5- und 1,8-Dinitronaphthalin entsteht, welches einen erhöhten Anteil an 1,5-Isomer enthält.

Überraschenderweise wurde nun gefunden, dass die Gegenwart von ionischen Flüssigkeiten bei der Nitrierung von Naphthalin in einem Überschuss an Salpetersäure oder Salpetersäure und Schwefelsäure und/oder Phosphorsäure eine Verschiebung des Isomerenverhältnisses hin zum 1,5-Dinitronaphthalin möglich ist.

Gegenstand der Erfindung ist somit ein Verfahren zur Herstellung eines Dinitronaphthalin-Isomerengemisches mit erhöhtem Anteil an 1,5-Dinitronaphthalin durch Nitrierung von Naphthalin mit einem molaren Überschuss an Salpetersäure, welches dadurch gekennzeichnet ist, dass die Nitrierung in Gegenwart mindestens einer ionischen Flüssigkeit durchgeführt wird.

Die erfindungsgemäß hergestellten Dinitronaphthalin-Isomerengemische enthalten neben 1,8-Dinitronaphthalin einen überraschend hohen Anteil an 1,5-Dinitronaphthalin. Bevorzugt liegt der Gehalt an 1,5-Dinitronaphthalin über 30 Gew.-%, bezogen auf den Umsatz, insbesondere bevorzugt zwischen 35 und 40 Gew.-%. Der Gehalt an Nebenprodukten, insbesondere 1,6- und 1,7-Dinitronaphthalin und höher nitrierten Produkten ist gering.

Im erfindungsgemäßen Verfahren wird Salpetersäure bevorzugt in einem 1 bis 22-fachen molaren Überschuss, besonders bevorzugt in einem 2 bis 20-fachen molaren Überschuss, ganz besonders bevorzugt in einem 4 bis 16-fachen molaren Überschuss, bezogen auf die in Naphthalin einzuführenden Nitrogruppen, eingesetzt.

Die Konzentration der im erfindungsgemäßen Verfahren eingesetzten Salpetersäure liegt bevorzugt zwischen

50 und 100 %, besonders bevorzugt zwischen 60 und 100 %, ganz besonders bevorzugt zwischen 67 und 98 %.

In einer weiteren Ausführungsform des erfindungsgemäßen Verfahrens kann die Salpetersäure in einer Mischung mit Schwefelsäure und/oder Phosphorsäure eingesetzt werden.

Wird im erfindungsgemäßen Verfahren Schwefelsäure eingesetzt, so liegt deren Konzentration bevorzugt zwischen 90 und 100 %, besonders bevorzugt zwischen 96 und 100 % und ganz besonders bevorzugt zwischen 98 und 100 %. Wird im erfindungsgemäßen Verfahren Phosphorsäure eingesetzt, so liegt deren Konzentration bevorzugt zwischen 50 bis 99 %, besonders bevorzugt zwischen 65 bis 99 % und ganz bevorzugt zwischen 85 und 99 %.

Wird im erfindungsgemäßen Verfahren eine Mischung aus Salpetersäure und Schwefelsäure und/oder Phosphorsäure eingesetzt, so besteht diese Mischung bevorzugt aus 1 bis 20 Teilen Salpetersäure und 1 Teil Schwefelsäure und/oder Phosphorsäure, besonders bevorzugt aus 1 bis 10 Teilen Salpetersäure und 1 Teil Schwefelsäure und/oder Phosphorsäure, ganz besonders bevorzugt aus 1 bis 5 Teilen Salpetersäure und 1 Teil Schwefelsäure und/oder Phosphorsäure.

Bei den im erfindungsgemäßen Verfahren eingesetzten ionischen Flüssigkeiten handelt es sich um flüssige Salze der Formel $Q^+ A^-$, die bevorzugt bei Temperaturen unterhalb von 90°C, insbesondere bei Temperaturen unterhalb von 80°C und besonders bevorzugt bei Temperaturen unterhalb von 50°C flüssige Salze bilden.

In der Formel $Q^+ A^-$ steht $Q^+$ bevorzugt für ein quarternäres Ammonium- und/oder Phosphoniumion. Besonders bevorzugt handelt es sich bei $Q^+$ um eine Verbindung aus der Reihe

$$R^1R^2R^3R^4N^+, R^1R^2R^3R^{4+}P, R^1R^2N^+{=}CR^3R^4, R^1R^2P^+{=}CR^3R^4$$

worin $R^1$ bis $R^4$ unabhängig voneinander Wasserstoff - mit Ausnahme von $NH_4^+$ -, gesättigtes oder ungesättigtes $C_1$-$C_{12}$-Alkyl, $C_3$-$C_8$-Cycloalkyl, $C_6$-$C_{10}$-Aryl oder $C_7$-$C_{11}$-Aralkyl bedeuten.

Besonders bevorzugt handelt es sich bei $Q^+$ um ein Ammonium- und/oder Phosphoniumion, welches sich von einem stickstoff- und/oder phosphorhaltigen Heterocyclus ableitet, welcher 1, 2 oder 3 Stickstoff- und/oder Phosphoratome aufweist und den nachfolgenden allgemeinen Formeln entspricht

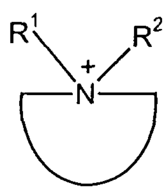
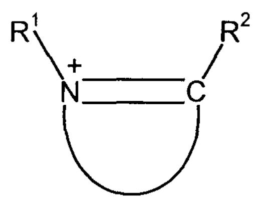
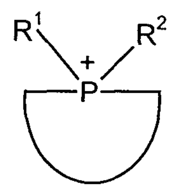
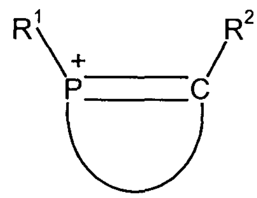

in welchen der Ring aus 4 bis 10, bevorzugt 5 bis 6 Atomen besteht und $R^1$ und $R^2$ die oben angegebene Bedeutung besitzen.

Weiterhin steht $Q^+$ bevorzugt für quarternäre Ammonium- und/oder Phosphoniumionen aus der Reihe

$$R^1R^2N^+{=}CR^3\text{-}R^5\text{-}R^3C{=}N^+R^1R^2 \text{ und } R^1R^2P^+{=}CR^3\text{-}R^5\text{-}R^3C{=}P^+R^1R^2$$

worin

$R^1$, $R^2$ und $R^3$ gleich oder verschieden sind und die oben angegebene Bedeutung besitzen und

$R^5$ für einen $C_1$-$C_6$-Alkylen- oder Phenylenrest steht.

Bevorzugte Beispiele für $R^1$ bis $R^4$ sind Methyl, Ethyl, Propyl, Isopropyl, Butyl, sek.-Butyl, tert.-Butyl, Amyl, Methylen, Ethyliden, Phenyl oder Benzyl, bevorzugte Beispiele für $R^5$ sind Methylen, Ethylen, Propylen oder Phenylen. In einer besonders bevorzugten Ausführungsform steht $Q^+$ für N-Butylpyridinium, N-Ethylpyridinium, 3-Butyl-1-methylimidazolium, Diethylpyrazolium, 3-Ethyl-1-methylimidazolium, Pyridinium, Tetramethylphenylammonium und Tetrabutylphosphonium. In einer ganz besonders bevorzugten Ausführungsform steht $Q^+$ für 3-Butyl-1-methylimidazolium, N-Butylpyridinium und N-Methylpyridinium.

In der Formel $Q^+ A^-$ steht $A^-$ bevorzugt für Hexafluorophosphat, Hexafluoroantimonat, Hexafluoroarsenat, Fluorosulfonat, Tetrafluoroborat, Nitrat, Alkylsulfonat oder Hydrogensulfat. Besonders bevorzugt steht $A^-$ für Tetrafluoroborat, Nitrat, Methylsulfonat, Propylsulfonat oder Hydrogensulfat.

Ganz besonders bevorzugte Verbindungen $Q^+ A^-$ sind 3-Butyl-1-methylimidazoliumtetrafluoroborat, 3-Butyl-1-methylimidazoliummethylsulfonat, 3-Butyl-1-methylimidazoliumpropylsulfonat, N-butylpyridiniummethylsulfonat und N-methylpyridiniumhydrogensulfat.

Im erfindungsgemäßen Verfahren können auch Mischungen verschiedener ionischer Flüssigkeiten eingesetzt werden.

Im erfindungsgemäßen Verfahren wird eine ionische Flüssigkeit oder Mischungen verschiedener ionischer Flüssigkeiten bevorzugt in einem 1 bis 10-fachen molaren Überschuss, besonders bevorzugt in einem 2 bis 6-fachen molaren Überschuss, ganz besonders bevorzugt in einem 2 bis 5-fachen molaren Überschuss, bezogen auf Naphthalin, eingesetzt.

Üblicherweise kann das erfindungsgemäße Verfahren bei Temperaturen von 50 bis 110°C, vorzugsweise bei Temperaturen von 60 bis 100°C und besonders bevorzugt bei Temperaturen von 80 bis 100°C durchgeführt werden.

Das erfindungsgemäße Verfahren kann ohne zusätzliches Lösungsmittel in einem Gemisch aus mindestens einer ionischer Flüssigkeit und Salpetersäure, gegebenenfalls in Anwesenheit von Schwefelsäure und/oder Phosphorsäure, durchgeführt werden. In einer weiteren Ausführungsform kann das erfindungsgemäße Verfahren auch nach Zusatz eines Lösungsmittels durchgeführt werden. Geeignete Lösungsmittel sind beispielsweise alle unter den Bedingungen der Nitrierung stabilen Lösungsmittel, bevorzugt Ligroin, Chloroform, Dichlormethan oder Cyclohexan.

Bevorzugt wird das erfindungsgemäße Verfahren ohne Zusatz eines Lösungsmittels durchgeführt.

Zur Durchführung des erfindungsgemäßen Verfahrens werden Naphthalin, mindestens eine ionische Flüssigkeit und die eingesetzte Säure vermischt. Um die vollständige Reaktion sicherzustellen, kann die Umsetzung unter guter Durchmischung des Reaktionsansatzes, beispielsweise durch intensives Rühren, durchgeführt werden. Die Reaktionsdauer liegt üblicherweise zwischen 30 Minuten und 20 Stunden, bevorzugt zwischen 2 und 18 Stunden. Die Aufarbeitung kann durch Einbringen des Reaktionsansatzes in Wasser und anschließender Extraktion mit organischem Lösungsmittel, bevorzugt mit einem nitrierten organischen Lösungsmittel, besonders bevorzugt mit Nitrobenzol, erfolgen. Wird zur Aufarbeitung des Reaktionsansatzes mit einem organischen Lösungsmittel extrahiert, so kann dieses durch dem Fachmann bekannte Maßnahmen wie Destillation entfernt werden.

Das Dinitronaphthalin-Isomerengemisch kann in bekannter Weise, beispielsweise durch fraktionierte Kristallisation, in die isomeren Dinitronaphthaline getrennt werden. Derartige Isomerentrennungen, beispielsweise mit Dimethylformamid oder Dichlorethan als Lösungsmittel, sind bereits beschrieben (Houben-Weyl, Methoden der organischen Chemie, 1971, Vol. 10, S. 494).

Die folgenden Beispiele sollen die Erfindung erläutern, ohne sie jedoch in ihrem Umfang zu begrenzen.

## Beispiele

### Beispiel 1

**Nitrierung von Naphthalin unter Verwendung von Salpetersäure in Gegenwart von N-Methylpyridiniumhydrogensulfat**

Zu 1 g 98 % (w/w) Salpetersäure (16 mmol) wurden 64 mg Naphthalin (0,5 mmol) und 0,5 g N-Methylpyridiniumhydrogensulfat (2,6 mmol) gegeben. Anschließend wurde der Ansatz 4 Stunden bei 100°C gerührt. Die Aufarbeitung erfolgte durch Einbringen des Reaktionsansatzes in 20 ml Eiswasser und anschließender Extraktion mit Nitrobenzol.

Die Isomerenzusammensetzung des Gemisches wurde gaschromatographisch bestimmt. Die angegebenen Werte sind effektive Gehalte, die Verbrennungsfaktoren der beiden Isomeren wurden berücksichtigt.

| | |
|---|---|
| 1,5 Dinitronaphthalin | 38% |
| 1,8 Dinitronaphthalin | 53% |
| Andere Dinitronaphthaline | 9,3% |
| Umsatz | 100% |

Die weiteren Beispiele 2 bis 9 wurden gemäß Beispiel 1 mit den in Tabelle 1 angegebenen Parametern durchgeführt.

**Tabelle 1**

| | Säure | Äquivalente Salpetersäure bezogen auf einzuführende Nitrogruppen | Ionische Flüssigkeit | Äquivalente ionische Flüssigkeit bezogen auf Naphthalin | Temperatur [°C] | t [h] | 1,5-Dinitronaphthalin [%] | 1,8-Dinitronaphthalin [%] | andere Dinitronaphthaline [%] | Verhältnis 1,5-Dinitronaphthalin zu 1,8-Dinitronaphthalin | Umsatz [%] |
|---|---|---|---|---|---|---|---|---|---|---|---|
| 1 | $HNO_3$ 98% | 16 | $MePyHSO_4$ | 5,0 | 100 | 4 | 38 | 53 | 9 | 0,72 | 100 |
| 2 | $HNO_3$ 98% | 8 | $MeBuImMeSO_3$ | 4,5 | 80 | 4 | 38 | 50 | 12 | 0,76 | 66 |
| 3 | $HNO_3$ 98% | 8 | $MeBuImPrSO_3$ | 4,0 | 100 | 16 | 38,5 | 52 | 10 | 0,74 | 74 |
| 4 | $HNO_3$ 98% | 8 | $MePyHSO_4$ | 5,0 | 100 | 2 | 36,5 | 51 | 12,5 | 0,72 | 94 |
| 5 | $HNO_3$ 80-98% | 22 | $MePyHSO_4$ | 5,0 | 100 | 2 | 39 | 51,5 | 9,5 | 0,76 | 89 |
| 6 | $HNO_3$ 67% $H_2SO_4$ 98% (2:1) | 12 | $MeBuImBF_4$ | 5,0 | 100 | 18 | 38,5 | 52 | 9,5 | 0,74 | 98 |
| 7 | $HNO_3$ 98% $H_2SO_4$ 98% (2 : 1) | 12 | $MeBuImBF_4$ | 4,5 | 100 | 18 | 38,5 | 52 | 9,5 | 0,74 | 97 |
| 8 | $HNO_3$ 98% $H_2SO_4$ 98% (2 : 1) | 12 | $MePyHSO_4$ | 5,0 | 100 | 4 | 37 | 50 | 13 | 0,73 | 75 |
| 9 | $HNO_3$ 98% $H_2SO_4$ 98% (2 : 1) | 12 | $MePyHSO_4$ | 5,0 | 100 | 16 | 38 | 50 | 12 | 0,76 | 87 |

mit $MeBuImMeSO_3$ = 3-Butyl-1-methylimidazoliummethylsulfonat
$MeBuImPrSO_3$ = 3-Butyl-1-methylimidazoliumpropylsulfonat
$MePyHSO_4$ = N-Methylpyridiniumhydrogensulfat
$MeBuImBF_4$ = 3-Butyl-1-methylimidazoliumtetrafluoroborat

**Patentansprüche**

1. Verfahren zur Herstellung eines Dinitronaphthalin-Isomerengemisches mit erhöhtem Anteil an 1,5-Dinitronaphthalin durch Nitrierung von Naphthalin mit einem molaren Überschuss an Salpetersäure, dadurch gekennzeichnet, dass die Nitrierung in Gegenwart mindestens einer ionischen Flüssigkeit durchgeführt wird.

2. Verfahren gemäß Anspruch 1, dadurch gekennzeichnet, dass es sich um einen 1 bis 22-fachen molaren Überschuss an Salpetersäure, bezogen auf die in Naphthalin einzuführenden Nitrogruppen, handelt.

3. Verfahren gemäß einem oder mehreren der vorangegangenen Ansprüche 1 und 2, dadurch gekennzeichnet, dass es sich bei der Salpetersäure um 50 bis 100 %ige Salpetersäure handelt.

4. Verfahren gemäß einem oder mehreren der vorangegangenen Ansprüche 1 bis 3, dadurch gekennzeichnet, dass zusätzlich Schwefelsäure und/oder Phosphorsäure eingesetzt wird.

5. Verfahren gemäß Anspruch 4, dadurch gekennzeichnet, dass es sich bei der Schwefelsäure um 90 bis 100 %ige Schwefelsäure und bei der Phosphorsäure um 50 %ige bis 99 %ige Phosphorsäure handelt.

6. Verfahren gemäß einem oder mehreren der vorangegangenen Ansprüche 4 und 5, dadurch gekennzeichnet, dass eine Mischung bezüglich molarer Anteile aus 1 bis 20 Teilen Salpetersäure und 1 Teil Schwefelsäure und/oder Phosphorsäure, insbesondere eine Mischung aus 5 bis 10 Teilen Salpetersäure mit 1 Teil Schwefelsäure und/oder Phosphorsäure eingesetzt wird.

7. Verfahren gemäß einem oder mehreren der vorangegangenen Ansprüche 1 bis 6, dadurch gekennzeichnet, dass es sich bei den ionischen Flüssigkeiten um Salze der Formel $Q^+ A^-$ handelt, welche bei einer Temperatur unterhalb von 90°C flüssig sind und worin

   $Q^+$ für ein quarternäres Ammonium- und/oder Phosphoniumion steht und

   $A^-$ für ein Anion aus der Reihe Hexafluorophosphat, Hexafluoroantimonat, Hexafluoroarsenat, Fluorosulfonat, Tetrafluoroborat, Nitrat, Alkylsulfonat oder Hydrogensulfat steht.

8. Verfahren gemäß Anspruch 7, dadurch gekennzeichnet, dass

   $Q^+$ für ein quarternäres Ammonium- und/oder Phosphoniumion aus der Reihe

$$R^1R^2R^3R^4N^+,\ R^1R^2N^+{=}CR^3R^4,\ R^1R^2R^3R^4P^+,\ R^1R^2P^+{=}CR^3R^4,$$

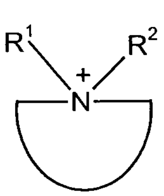 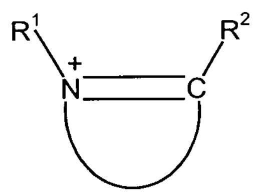 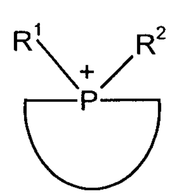 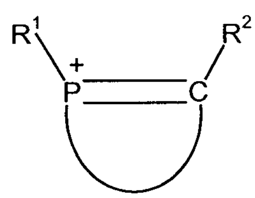

   steht, wobei

   $R^1$, $R^2$, $R^3$ und $R^4$ gleich oder verschieden sind und für Wasserstoff - mit Ausnahme von $NH_4^{+}$ - gesättigtes oder ungesättigtes $C_1$-$C_{12}$-Alkyl, $C_3$-$C_8$-Cycloalkyl, $C_6$-$C_{10}$-Aryl oder $C_7$-$C_{11}$-Aralkyl stehen und die Ringe aus 4 bis 10 Atomen bestehen.

9. Verfahren gemäß einem oder mehreren der vorangegangenen Ansprüche 1 bis 8, dadurch gekennzeichnet, dass es sich bei den ionischen Flüssigkeiten um 3-Butyl-1-methylimidazoliumtetrafluoroborat, 3-Butyl-1-methylimidazoliummethylsulfonat, N-Butylpyridiniummethylsulfonat, 3-Butyl-1-methylimidazoliumpropylsulfonat oder N-Methylpyridiniumhydrogensulfat handelt.

**10.** Verfahren gemäß einem oder mehreren der vorangegangenen Ansprüche 1 bis 9, dadurch gekennzeichnet, dass man die ionische Flüssigkeit in einer 1- bis 10-fachen molaren Menge, bezogen auf die Menge an Naphthalin, einsetzt.

Europäisches Patentamt

# EUROPÄISCHER RECHERCHENBERICHT

Nummer der Anmeldung
EP 00 12 5282

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,Y | DE 11 50 965 B (BADISCHE ANILIN- & SODA-FABRIK AG) 4. Juli 1963 (1963-07-04)<br>* das ganze Dokument *<br>--- | 1-10 | C07C205/06<br>C07C201/08 |
| P,Y | WO 00 32572 A ( SYMYX TECHNOLOGIES) 8. Juni 2000 (2000-06-08)<br>* Seite 24; Ansprüche 1-10 *<br>--- | 1-10 | |
| E | WO 01 09065 A (MERCK PATENT GMBH) 8. Februar 2001 (2001-02-08)<br>* Seite 8 - Seite 9; Anspruch 1 *<br>----- | 1,7,8 | |

RECHERCHIERTE SACHGEBIETE (Int.Cl.7)
C07C

Der vorliegende Recherchenbericht wurde für alle Patentansprüche erstellt

| Recherchenort | Abschlußdatum der Recherche | Prüfer |
|---|---|---|
| DEN HAAG | 23. März 2001 | Bonnevalle, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 00 12 5282

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-03-2001

| Im Recherchenbericht angeführtes Patentdokument | Datum der Veröffentlichung | Mitglied(er) der Patentfamilie | Datum der Veröffentlichung |
|---|---|---|---|
| DE 1150965 B | | KEINE | |
| WO 0032572 A | 08-06-2000 | AU 2165000 A | 19-06-2000 |
| WO 0109065 A | 08-02-2001 | DE 19935692 A | 01-02-2001 |

EPO FORM P0461

Für nähere Einzelheiten zu diesem Anhang : siehe Amtsblatt des Europäischen Patentamts, Nr.12/82